# EUROPÄISCHE PATENTANMELDUNG

(11) **EP 3 320 946 A1**
(43) Veröffentlichungstag der Anmeldung: **16.05.2018**
(21) Anmeldenummer: 16198876.1
(22) Anmeldetag: 15.11.2016
(51) Int. Cl.: A61N 1/05

(54) **IMPLANTIERBARE LEITUNG**

(71) Anmelder: BIOTRONIK SE & Co. KG, 12359 Berlin (DE)
(72) Erfinder: Täubert, Kerstin, 12589 Berlin (DE); Kolberg, Gernot, 12161 Berlin (DE)
(74) Vertreter: Galander, Marcus

(57) **Zusammenfassung**

Implantierbare Leitung, die einen Elektrodenkörper mit einem freien Ende und ein nahe dem freien Ende im Elektrodenkörper angeordnetes und diesem gegenüber axial verschiebliches oder drehverschiebliches Innenteil aufweist, das ein zum freien Ende des Elektrodenkörpers benachbartes Ende hat, an dem ein durch die Axialverschiebung des Innenteils aus dem freien Ende des Elektrodenkörpers ausfahrbares Fixierungsmittel zur Fixierung der Leitung an Körpergewebe im implantierten Zustand angebracht ist, wobei am Innenumfang des Elektrodenkörpers ein elastisch deformierbares umfängliches, insbesondere ring- oder ringsegmentförmiges Dicht- und/oder Widerstandselement, fixiert und am Außenumfang des Innenteils ein Abschnitt mit sich in axialer Richtung ändernden, insbesondere zum freien Ende des Elektrodenkörpers hin verringerndem Durchmesser vorgesehen ist, welcher bezüglich des Dicht- und Widerstandselementes derart platziert ist, dass er bei der Axialverschiebung des Innenteils das Dicht- und Widerstandselement passiert, oder wobei am Außenumfang des Innenteils ein elastisch deformierbares ring-oder ringsegmentförmiges Dicht- und Widerstandselement fixiert und am Innenumfang des Elektrodenkörpers ein Abschnitt mit sich in axialer Richtung ändernden, insbesondere zum freien Ende des Elektrodenkörpers hin verringerndem Durchmesser vorgesehen ist, welcher bezüglich des Dicht- und Widerstandselementes derart platziert ist, dass das Dicht-und Widerstandselement ihn bei der Axialverschiebung des Innenteils passiert, wobei sich das Dicht- und Widerstandselement bei der Axialverschiebung des Innenteils zunehmend verformt und der Bewegung des Innenteils und somit dem Ausfahren des Fixierungsmittels zunehmenden Widerstand entgegensetzt.

## Beschreibung

### Gebiet der Erfindung

Die Erfindung betrifft eine implantierbare Leitung, die einen Elektrodenkörper mit einem freien Ende und ein im Elektrodenkörper angeordnetes und diesem gegenüber axial verschiebliches Innenteil aufweist, das ein zum freien Ende des Elektrodenkörpers benachbartes Ende hat, an dem ein durch die Axialverschiebung des Innenteils aus dem freien Ende des Elektrodenkörpers ausfahrbares Fixierungsmittel zur Fixierung der Leitung an Körpergewebe im implantierten Zustand angebracht ist.

### Stand der Technik

Derartige Leitungen sind insbesondere als Elektrodenleitungen von Schrittmacher- oder Kardioverteranordnungen seit langem bekannt und in breitem klinischen Einsatz. Dies ist auf erhebliche Vorteile dieses Leitungstyps zurückzuführen, wie etwa die besonders sichere Fixierung am zu stimulierenden Herzgewebe und hierdurch erzielte hohe Zuverlässigkeit der Gesamtanordnung sowie den relativ einfachen Aufbau und die gute Handhabbarkeit für den Operateur. Besonders verbreitet ist hierbei der Leitungstyp, bei dem das Fixierungsmittel als Schraubhelix ausgeführt ist, die aus dem Ende der Leitung heraus und zugleich in das anstoßende Herzgewebe hinein geschraubt und im Falle einer erforderlichen Repositionierung mit umgekehrtem Drehsinn auch wieder aus dem Herzgewebe heraus geschraubt und in das Leitungsende zurückgezogen werden kann. Rein beispielhaft wird zu diesem Stand der Technik verwiesen auf die aktuelle US 9,302,098 B2.

Gleichwohl haben die bekannten gattungsgemäßen Leitungen auch Nachteile. Einfache Leitungskonstruktionen mit feststehender, bereits im Lieferzustand aus dem Leitungsende vorstehender Fixierungshelix müssen durch einen auflösbaren Schutzmantel am Leitungsende während der Implantation bedeckt sein. Diese Konstruktionen sind in ihrem zeitlichen Verhalten während der Implantation für den Operateur nicht ohne weiteres berechenbar, und es besteht das Risiko einer zu frühen Auflösung des Schutzmantels und von damit verbundenen Problemen bei der Einführung der Leitung, ebenso wie einer zu späten Auflösung und einer hierdurch bedingten ungenügenden Fixierung der Leitung.

### Aufgabe der Erfindung

Der Erfindung liegt daher die Aufgabe der Bereitstellung einer verbesserten Leitung der gattungsgemäßen Art zugrunde, die insbesondere leicht handhabbar, zuverlässig und ohne Risiken während der Einführung implantierbar und zuverlässig im Langzeiteinsatz sein soll.

Diese Aufgabe wird durch eine implantierbare Leitung mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, die Leitung so aufzubauen, dass beim Ausfahren des distal angeordneten Fixierungsmittels ein in Abhängigkeit von dessen Position bezüglich des Leitungsendes veränderlicher Grad an Leicht- bzw. Schwergängigkeit erreicht wird. In der Anfangsphase soll nach den Überlegungen der Erfinder die Ausfahrbewegung sehr leichtgängig, in der Endphase jedoch deutlich schwergängiger sein. Die anfangs leichtgängige Fixierung soll im ausgeschraubten Endzustand so in der Drehbarkeit bzw. Axial-Verschieblichkeit gehemmt bzw. festgehalten werden, dass ein ungewolltes Zurückschrauben erschwert bzw. vermieden wird. Weiterhin schließt die Erfindung den Gedanken ein, innerhalb der Leitung ein elastisch deformierbares Dichtelement derart auszubilden, dass es zugleich als Widerstandselement funktioniert und beim Ausschrauben des Fixierungsmittels deformiert wird, wobei sich der der weiteren Bewegung entgegengesetzte Widerstand erhöht.

Weiterhin schließt die Erfindung den Gedanken ein, dass dieses Dicht- und/oder Widerstandselement am Innenumfang des Elektrodenkörpers feststehend angeordnet ist und dass am Außenumfang eines im Elektrodenkörper verschieblichen oder drehverschieblichen Innenteils ein Abschnitt mit in axialer Richtung veränderlichem Durchmesser vorgesehen ist. Dieser Abschnitt ist bezüglich der Position des Dicht- und Widerstandselementes derart platziert, dass er bei der Axialverschiebung des Innenteils das umfängliche, spezielle ring- oder ringsegmentförmige Dicht- und Widerstandselement passiert. In einer alternativen Konfiguration ist das Dicht- und Widerstandselement am Außenumfang des Innenteils (und somit bezüglich des Elektrodenkörpers verschieblich) angeordnet, und es ist am Innenumfang des Elektrodenkörpers ein Abschnitt mit veränderlichem Durchmesser vorgesehen, der derart positioniert ist, dass das Dicht- und Widerstandselement ihn bei der Axialverschiebung des Innenteils passiert.

In einem ersten, aus derzeitiger Sicht praktisch besonders wichtigen Anwendungsfall ist die vorgeschlagene Leitung ausgebildet als Elektrodenleitung zum Anschluss an ein elektromedizinisches Gerät, mit mindestens einem Elektrodenpol und mindestens einer dem Innenteil zugeordneten elektrischen Zuleitung.

Es ist aber auch ein Einsatz als Katheterleitung, also ohne Elektrodenpol(e) und entsprechende Zuleitung(en), in anderen Einsatzgebieten als demjenigen der Elektrostimulation möglich. Beispielsweise könnte sich die vorgeschlagene Leitung auch für die korrekte und zuverlässige Positionierung bestimmter Sensoren für das intrakorporale Abfühlen von Körperfunktionsparametern oder auch für die präzise lokale Zuführung von Fluiden eignen.

In einer aus derzeitiger Sicht bevorzugten Konstruktion ist das Innenteil im Elektrodenkörper drehverschieblich gehaltert, derart, dass das Fixierungsmittel durch überlagerte Axialverschiebung und Rotation aus dem freien Ende des Elektrodenkörpers ausfahrbar ist. In einer weiter bevorzugten Ausgestaltung ist das Fixierungsmittel eine Schraubhelix. Grundsätzlich ist die vorgeschlagene Leitung aber auch mit andersartigen Fixierungsmitteln ausführbar, wie etwa mit einer Gruppe ausspreizbarer Fixierungshaken o.ä..

In kostengünstigen und zuverlässig wirkenden konstruktiven Ausgestaltungen ist das Dicht- und Widerstandselement ein Ring aus kompressiblem Material, welcher beim Durchgang des oben erwähnten Abschnitts mit veränderlichem Durchmesser im Zuge des Ausfahrens des Fixierungsmittels zunehmend komprimiert wird. Die zunehmende Kompression kann etwa dadurch erzielt werden, dass der kompressible Ring zwischen zwei Ringflansche an seinem Träger - also dem äußeren Elektrodenkörper oder dem Innenteil - eingespannt ist und somit seitlich nicht "ausweichen" kann.

In alternativen Ausführungen ist ein Ring (oder auch eine Gruppe von Ringsegmenten) aus einem elastisch deformierbaren Material vorgesehen, welches derart geometrisch konfiguriert ist, dass der Ring sich beim Passieren des Abschnitts mit veränderlichem Durchmesser des jeweiligen Leitungs-Gegenstücks zunehmend deformiert und die Widerstandserhöhung aus der zunehmenden Deformation resultiert. Diese Ausführungen können auch mit den Vorgenannten kombiniert sein.

Wie bereits oben angemerkt, muss das Dicht- und Widerstandselement nicht notwendigerweise eine geschlossene Ringform haben, sondern es kann auch aus einer Gruppe von Ringsegmenten gebildet sein, und die Widerstandsfunktion dieses Funktionselementes kann z. B. sogar durch einzelne Reibblöcke realisiert werden. Um mit geringem konstruktivem Aufwand sowohl die im Rahmen der Erfindung angestrebte Widerstandselement-Funktion als auch die herkömmlicherweise erforderliche Dichtelement-Funktion erreichen zu können, ist im Hinblick auf die zylindrische Gestalt des Elektrodenkörpers und üblicherweise ebenso zylindrisch gestaltetes Innenteils die Ringform jedoch als bevorzugt anzusehen.

In zweckmäßigen materialseitigen Ausführungen weist das Dicht- und Widerstandselement ein Silikon- oder Polyurethanmaterial auf. Geeignet sind jedoch auch andere hinreichend kompressible und/oder elastische biokompatible Materialien.

In einer Fortentwicklung des Konzepts der Erfindung ist vorgesehen, dass in axialer Richtung der Leitung am Elektrodenkörper oder Innenteil auf den Abschnitt sich ändernden Durchmessers mindestens einseitig ein Abschnitt konstanten Durchmessers folgt, dessen Durchmesser gleich dem kleinsten bzw. dem größten Durchmesser des Abschnitts mit sich änderndem Durchmesser ist. Einzelheiten und Vorteile entsprechender Konstruktionen werden weiter unten angegeben.

In einer weiteren konstruktiven Ausgestaltung ist an oder nahe einem Ende des Abschnitts mit sich verringerndem Durchmesser ein ringförmiger Einwölbungsabschnitt angeordnet, in den das Dicht- und Widerstandselement beim Ausfahren des Fixierungsmittels und/oder beim Wiedereinziehen des Fixierungsmittels hineingleitet sich teilweise rück-verformt und einer weiteren axialen Bewegung des Innenteils bezüglich des Elektrodenkörpers einen sprunghaft erhöhten Widerstand entgegensetzt. Auch hierzu finden sich Einzelheiten in den Erläuterungen von Ausführungsbeispielen weiter unten.

In einer zweckmäßigen geometrischen Konfiguration des Innenteils bzw. des Elektrodenkörpers ist der zugeordnete Abschnitt mit sich änderndem Durchmesser konisch ausgebildet. Alternativ hierzu kann vorgesehen sein, dass der Abschnitt mit sich änderndem Durchmesser eine sich stufig oder bogenförmig verjüngende Oberfläche aufweist.

In einer weiteren Ausführung der Erfindung ist vorgesehen, dass das Innenteil oder der Elektrodenkörper mehrere Abschnitte mit sich änderndem, insbesondere mit sich in entgegengesetzten Richtungen verringerndem, Durchmesser und/oder mit unterschiedlichem Kegelwinkel, aufweist.

In weiteren Ausführungen der Erfindung ist auf mindestens einem Abschnitt des Innenteils oder der Innenwandung des Elektrodenkörpers eine Beschichtung mit vorbestimmtem Reibwert gegenüber dem Dicht- und Widerstandselement zur selektiven Vergrößerung oder Verringerung des der axialen Verschiebung oder Drehverschiebung des Innenteils entgegengesetzten Widerstandes vorgesehen. In einer Ausgestaltung dieser Ausführung sind auf verschiedenen Abschnitten des Innenteils oder der Innenwandung des Elektrodenkörpers Beschichtungen mit unterschiedlichem Reibwert gegenüber dem Dicht- und Widerstandselement vorgesehen.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1A und 1B: schematische Längsschnittdarstellungen einer implantierbaren Leitung gemäß einer ersten Ausführungsform der Erfindung im zurückgezogenen bzw. ausgefahrenen Zustand Ihres Fixierungsmittels,
- Fig. 2A und 2B: schematische Längsschnittdarstellungen einer implantierbaren Leitung gemäß einer zweiten Ausführungsform der Erfindung im zurückgezogenen bzw. ausgefahrenen Zustand Ihres Fixierungsmittels,
- Fig. 3A und 3B: schematische Längsschnittdarstellungen einer implantierbaren Leitung gemäß einer dritten Ausführungsform der Erfindung im zurückgezogenen bzw. ausgefahrenen Zustand Ihres Fixierungsmittels,
- Fig. 4: eine schematische Längsschnittdarstellung einer implantierbaren Leitung gemäß einer vierten Ausführungsform der Erfindung im zurückgezogenen Zustand Ihres Fixierungsmittels,
- Fig. 5: eine schematische Längsschnittdarstellung einer implantierbaren Leitung gemäß einer fünften Ausführungsform der Erfindung im zurückgezogenen Zustand Ihres Fixierungsmittels, und
- Fig. 6: eine schematische Längsschnittdarstellung einer implantierbaren Leitung gemäß einer sechsten Ausführungsform der Erfindung im zurückgezogenen Zustand Ihres Fixierungsmittels.

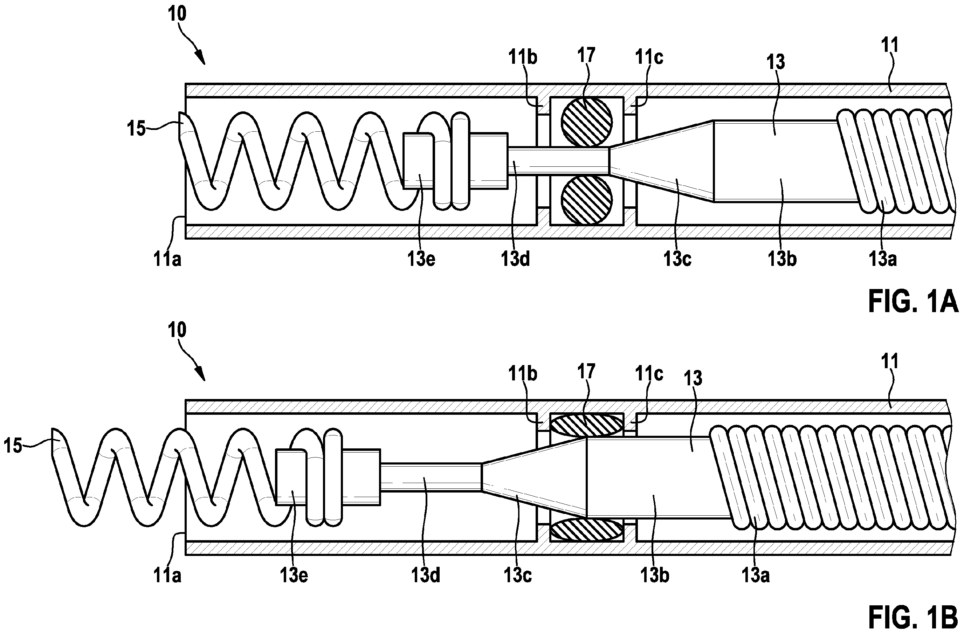

Fig. 1A und 1B zeigen in Form einer schematischen Längsschnittdarstellung den distalen Endabschnitt einer implantierbaren Elektrodenleitung 10, bei der in einem im Wesentlichen hohlen, zylindrischen Elektrodenkörper 11 ein drehverschiebliches Innenteil 13 angeordnet ist. Das Innenteil 13 trägt an seinem distalen Ende, welches dem freien (distalen) 11a des Elektrodenkörpers 11 benachbart ist, eine Schraubhelix 15 zur Fixierung der Leitung an Körpergewebe, speziell Herzgewebe, eines Patienten.

Beabstandet vom freien Ende 11a sind am Innenumfang des Elektrodenkörpers 11 zwei voneinander beabstandete Ringflansche 11b, 11c angeformt, zwischen denen ein kompressibel verformbarer Dichtring 17 gehaltert ist. Das Innenteil 13 hat, von proximal nach distal in dieser Reihenfolge, einen Wendelabschnitt 13a, einen ersten zylindrischen Abschnitt 13b, einen sich nach distal konisch verjüngenden (kegelstumpfförmigen) Abschnitt 13c, einen zweiten zylindrischen Abschnitt 13 und einen in seiner Grundform ebenfalls zylindrischen Schraubhelix-Befestigungsabschnitt 13e. Der konische Abschnitt 13c hat einen kleinsten Durchmesser, der gleich dem Durchmesser des distal angrenzenden zweiten zylindrischen Abschnitts 13d ist, und einen größten Durchmesser, der gleich dem Durchmesser des proximal angrenzenden zylindrischen Abschnitts 13b ist.

Das Innenteil 13 ist über ein Betätigungselement an seinem (nicht gezeigten) proximalen Ende mittels des Wendelabschnitts 13a gegenüber dem Elektrodenkörper 11 drehverschieblich, wodurch es von dem in Fig. 1A gezeigten Ausgangs- bzw. Lieferzustand in den in Fig. 1B gezeigten Gebrauchszustand versetzt wird, wobei die Schraubhelix 15 aus dem freien Leitungsende 11a ausgefahren und in (nicht gezeigtem) anstoßendem Körpergewebe des Patienten schraub-fixiert wird. Während der axialen Drehverschiebung des Innenteils und dem hiermit verbundenen Ausfahren der Schraubhelix passiert der konische Abschnitt 13c des Innenteils die Ringflansche 11b, 11c am Elektrodenkörper und den zwischen diesen gehalterten Dichtring 17 und deformiert diesem von der in Fig. 1A gezeigten Anfangsform in die in Fig. 1B gezeigte Endform. Durch die Vergrößerung der Kontaktfläche bzw. Erhöhung der axialen Druckspannung des Dichtringes 17 mit dem Innenteil 11 und die Kompression des Dichtringes vergrößert sich graduell der durch den Dichtring ausgeübte Widerstand auf die weitere Axialverschiebung des Innenteils. In jedem Zustand ist der Bereich proximal des Dichtringes gegenüber dem Bereich distal des Dichtringes, also insbesondere gegenüber dem Eindringen von Körperflüssigkeit im implantierten Zustand, abgedichtet, die Axialbewegung des Innenteils ist jedoch in der Anfangsphase erheblich (und für den Operateur auch fühlbar) leichtgängiger als in der Endphase, kurz vor Erreichen des voll ausgefahrenen Zustandes der Schraubhelix 15.

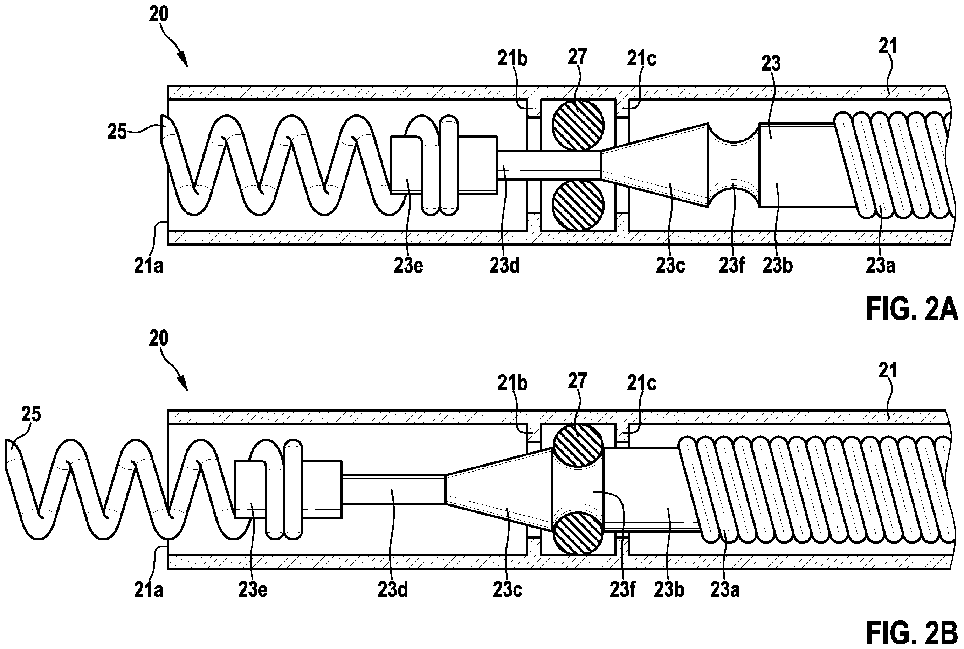

Fig. 2A und 2B zeigen in gleicher Weise wie Fig. 1A und 1B als weiteres Ausführungsbeispiel eine implantierbare Leitung 20. Deren Aufbau ist weitgehend der gleiche wie beim ersten Ausführungsbeispiel, so dass einander entsprechende Komponenten mit korrespondierenden Bezugsziffern bezeichnet sind und hier nicht weiter erläutert werden. Die Leitung 20 unterscheidet sich von der oben beschriebenen Leitung 10 dadurch, dass das Innenteil 23 zwischen dem ersten zylindrischen Abschnitt 23b und dem konischen bzw. kegelstumpfförmigen Abschnitt 23c eine ringförmige Vertiefung 23f (Ringnut) mit annähernd kreisabschnittsförmigem Querschnitt aufweist.

Diese Ringnut 23f ist so positioniert und konfiguriert, dass bei Erreichen des vollständig ausgefahrenen Zustandes der Schraubhelix 25 das zwischen den Ringflanschen 21b, 21c eingeklemmte Dicht- und Widerstandselement 27 hinein gleitet und sich dabei partiell rück-verformt und dekomprimiert. Dies führt zu einer haptisch wahrnehmbaren Änderung des durch das Dicht- und Widerstandselement dem Herausfahren der Schraubhelix entgegengesetzten Widerstandes und signalisiert dem Operateur somit die Erreichung des voll ausgeschraubten Zustandes dieses Fixierungsmittels. Die ansonsten nur durch einen Röntgenkontrastmarker mögliche Signalisierung dieses Zustandes ist mithin auch auf andere Weise realisierbar oder zumindest verifizierbar. Außerdem erschwert das stabile Ruhen des partiell rück-verformten Dichtringes 27 in der Ringnut 23f ein unbeabsichtigtes Zurückfahren der Schraubhelix in das offene Ende der Leitung 20 und somit ein unbeabsichtigtes Lösen der Fixierung der Leitung am Körpergewebe, oder es kann dieses sogar verhindern.

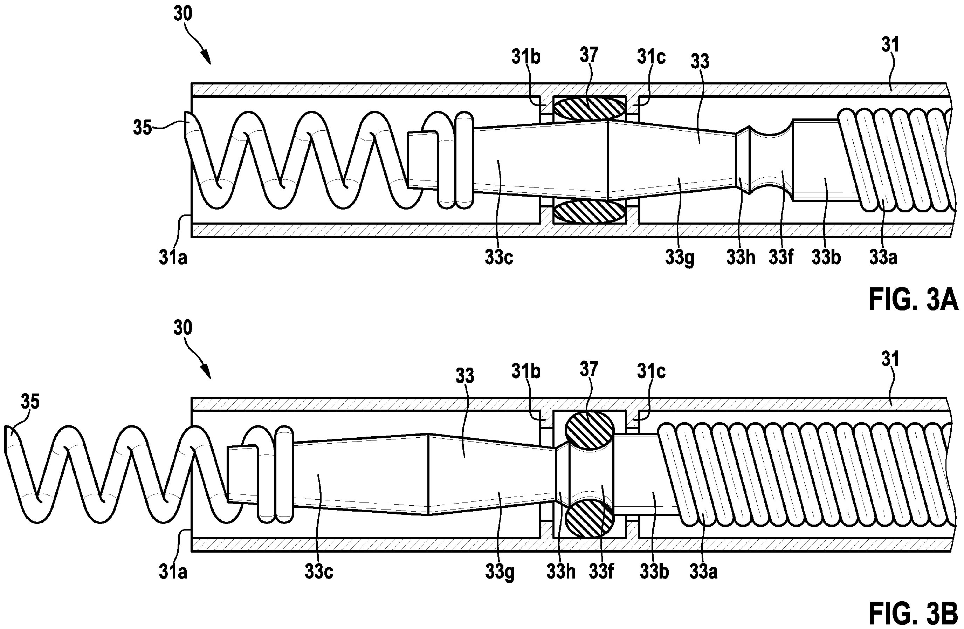

Fig. 3A und 3B zeigen in einer wiederum zu Fig. 1A/1B und Fig. 2A/2B ähnlichen Darstellungsweise als weiteres Ausführungsbeispiel eine implantierbare Leitung 30. Insoweit deren Aufbau dem Aufbau der weiter oben beschriebenen Leitungen 10 und 20 entspricht, sind die einzelnen Teile mit korrespondierenden Bezugsziffern versehen und werden hier nicht nochmals beschrieben.

Die Leitung 30 unterscheidet sich von den Leitungen 10 und 20 durch eine wesentlich komplexere geometrische Konfiguration des Innenteils 33, das hier in seinem distalen Endabschnitt 33c durchgehend leicht konisch (mit kleinem Kegelwinkel) zum freien Ende 31 a der Leitung verjüngend geformt ist, in proximaler Richtung hieran anschließend einen ebenfalls leicht konischen, sich jedoch in proximaler Richtung verjüngenden Abschnitt 33g, anschließend einen stark konischen Verdickungsabschnitt 33h und schließlich, vor dem Übergang zum proximalen zylindrischen Teil 33a, eine Ringnut 33f umfasst. Der distale, leicht konische Abschnitt 33c kann auch zylindrisch ausgebildet sein.

Die wesentlichen Wirkungen dieses Aufbaus des Innenteils sind wie folgt: Wenn beim Übergang vom in Fig. 3A gezeigten Anfangszustand in den in Fig. 3B gezeigten Endzustand (implantierten Zustand) der sich in proximaler Richtung leicht verjüngende Abschnitt 33g des Innenteils 33 das Dicht- und Widerstandselement 37 passiert, kann dieses sich graduell ausdehnen, so dass die Verschiebung des Innenteils gegenüber dem Elektrodenkörper 31 umso leichtgängiger wird, je weiter die Schraubhelix 35 ausgefahren ist, und zwar bis zum Übergangspunkt zum Verdickungsabschnitt 33h. Dort erfolgt eine sprunghafte Kompression des Dicht- und Widerstandselementes 37, die sich als fühlbare Widerstandserhöhung manifestiert, bevor das Dicht- und Widerstandselement 37 schließlich in die Ringnut 33f gleitet und sich wieder etwas ausdehnt.

Diese Konfiguration erlaubt eine noch deutlichere Wahrnehmung des nahenden und erreichten Endzustandes des Ausfahrens der Schraubhelix (des Fixierungsmittels) 35. Wie bei der Leitung 20, bewirkt das "Verrasten" des Dicht- und Widerstandselementes 37 in der Ringnut 33f des Innenteils zudem eine weitgehende selbsttätige Arretierung des Innenteils und somit der Schraubhelix gegenüber dem Elektrodenkörper im ausgefahrenen Zustand. Zusätzlich wird, sollte diese erste Hemmung bzw. Arretierung doch einmal überwunden werden, einem weiteren unbeabsichtigten Zurückfahren bzw. Zurückdrücken des Fixierungsmittels in die Leitung (beispielsweise durch äußere Krafteinwirkungen) durch die revers konische Gestalt des Abschnitts 33g des Innenteils ein zunehmender Widerstand entgegengesetzt.

Dieser revers konische (d. h. sich in proximaler Richtung verjüngende) Abschnitt 33g hat überdies die Wirkung, dass das Innenteil 33 gewissermaßen "von selbst" also ohne wesentlichen Betätigungsaufwand, aus dem Elektrodenkörper herausfahren kann und die Schraubhelix sich somit auch weitgehend selbsttätig in das Körpergewebe einschraubt, an dem die Leitung zu fixieren ist.

Ist der distal vom Abschnitt 33g angeordnete Abschnitt 33c zum freien Ende hin leicht verjüngend ausgeführt und durchläuft auch dieser Abschnitt bei der Drehverschiebung des Innenteils gegenüber dem Elektrodenkörper das Dicht- und Widerstandselement 37, ergibt sich eine zusätzliche Stufe der Ausfahrbewegung, nämlich eine erste Stufe mit sich graduell etwas erhöhendem Widerstand, bevor der Umkehrpunkt der Verjüngung erreicht wird und die zweite Bewegungsphase mit sich graduell verringerndem Widerstand folgt.

Aus obigem ergibt sich, dass sich durch einen komplexen Aufbau des Innenteils mit mehreren konischen Abschnitten und/oder Ringnuten weitgehend gewünschte axiale Widerstandsverläufe beim Herausfahren des Fixierungsmittels aus einer erfindungsgemäßen Leitung erzielen lassen.

Lediglich beispielhaft, ohne genauere Beschreibung, wird hier noch eine Konfiguration erwähnt, bei der eine Ringnut an einer solchen Stelle des Innenteils vorgesehen ist, dass das Dicht- und Widerstandselement am Elektrodenkörper im vollständig eingefahrenen Zustand des Innenteils (also im Lieferzustand der Leitung) in ihr ruht. Hierdurch wird sicher verhindert, dass sich das Innenteil bereits vor dem Implantationsvorgang, also beispielsweise während des Transports, gegenüber dem Elektrodenkörper vierschiebt und das Fixierungsmittel unbeabsichtigt aus dem Leitungsende austritt.

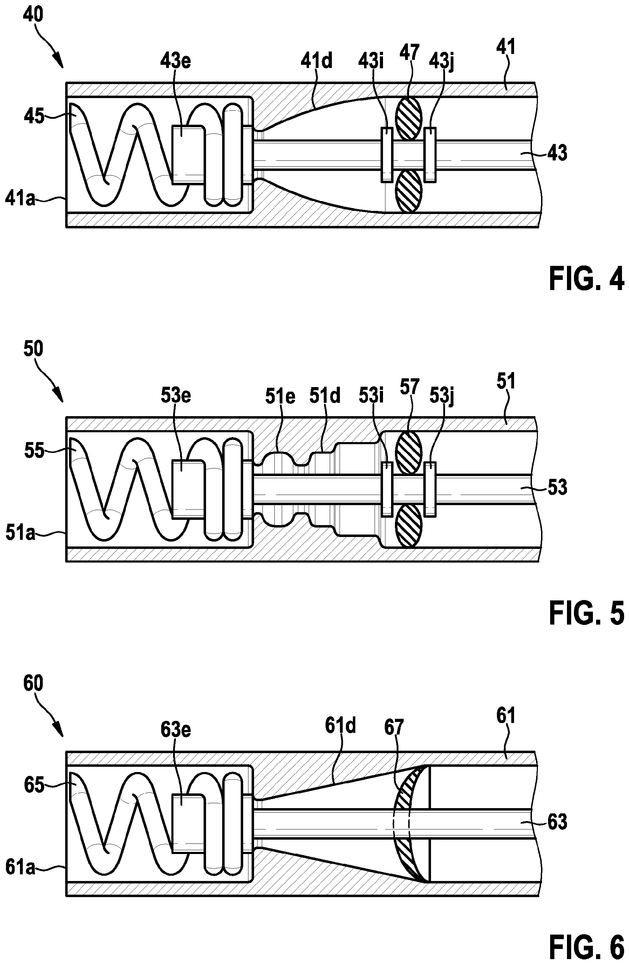

Fig. 4 zeigt in einer schematischen Längsschnittdarstellung als weiteres Ausführungsbeispiel eine implantierbare Leitung 40, bei der die Zuordnung wesentlicher Teile bzw. Abschnitte gegenüber den oben beschriebenen Ausführungen nach Fig. 1A/1B bis 3A/3B umgekehrt ist. Wiederum sind gleiche oder funktionsähnliche Komponenten mit an die Figuren 1A/1B bis 3A/3B angepassten Bezugsziffern bezeichnet und werden hier nicht nochmals im Einzelnen beschrieben.

Bei der Leitung 40 ist ein Dichtring (Dicht- und Widerstandselement) 47 aus kompressibel verformbaren Material (etwa einem PU-Kunststoff) zwischen zwei Ringflanschen 43i, 43j auf dem Innenteil 43 angebracht, welches hier bis auf den distalen Befestigungsabschnitt 43e für eine Schraubhelix 45 durchgehend zylindrisch ist. Jedoch ist bei dieser Leitung 40 der Elektrodenkörper 41 mit einem Verjüngungsabschnitt 41d versehen, in welchem sich der Innendurchmesser des Elektrodenkörpers nach distal bogenartig progressiv verringert. Beim drehenden Verschieben des Innenteils 43 gegenüber dem Elektrodenkörper 41 und dem hiermit bewirkten Herausfahren der Schraubhelix 45 durchläuft das Dicht- und Widerstandselement 47 diesen Abschnitt 41d mit sich progressiv verringerndem Durchmesser, wird hierbei progressiv verformt und komprimiert und setzt aufgrund dessen der weiteren Bewegung einen sich progressiv erhöhenden Widerstand entgegen.

Fig. 5 zeigt als weiteres Beispiel eine Leitung 50, deren Aufbau weitgehend demjenigen der Leitung 40 in Fig. 4 entspricht und deren Komponenten folglich mit an Fig. 4 angepassten Bezugsziffern versehen sind. Die Leitung 50 unterscheidet sich von der Leitung 40 in einer anderen geometrischen Konfiguration des Elektrodenkörpers 51. Dieser umfasst hier einen Verjüngungsabschnitt 51d mit sich stufig verringerndem Innendurchmesser und, distal hieran anschließend, eine Ringnut 51e mit annähernd kreisabschnittsförmigem Querschnitt.

Der stufige Abschnitt 51d übt beim Durchfahren durch das Innenteil 53 mit dem daran fixierten Dicht- und Widerstandselement 57 eine stufenartig zunehmende Kompression und Verformung auf das Dicht- und Widerstandselement aus, was zu einem ebenfalls stufenartigen Ansteigen des der weiteren Bewegung entgegengesetzten Widerstandes führt. Diese Widerstands-Stufen sind für den Operateur fühlbar und signalisierten ihm jeweils eine Stufe des Herausfahrens der Schraubhelix 55 aus dem Leitungsende 51a. Ähnlich wie bei der Leitung 20 nach Fig. 2A/2B gleitet das Dicht- und Widerstandselement am Ende der Ausfahrbewegung der Schraubhelix in die Ringnut 51e und dehnt sich wieder etwas aus, was zu einer sprunghaften Verringerung des Widerstandes führt und dem Operateur für die Beendigung des Ausfahr- und Fixierungsvorganges signalisiert.

Fig. 6 zeigt als weiteres Beispiel eine implantierbare Leitung 60, deren Aufbau weitgehend demjenigen der Leitung 40 nach Fig. 4 entspricht, so dass auch hier korrespondierende Bezugsziffern vergeben wurden und weiter oben gegebene Erläuterungen nicht wiederholt werden.

Ein wesentlicher Unterschied zur Leitung 40 besteht darin, dass der Verjüngungsabschnitt 61 d am Innenumfang des Elektrodenkörpers 61 konisch, d. h. kegelstumpfförmig, ist. Ein weiterer wesentlicher Unterschied besteht darin, dass das am Innenteil angebrachte Dicht- und Widerstandselement 67 als korbartiges, weich elastisches Element ohne wesentliche Kompressibilität, etwa aus einem Silikonmaterial, ausgeführt ist. Beim fortschreitenden Verschieben des Innenteils 63 zum offenen Ende 61a der Leitung hin wird das Dicht- und Widerstandselement 67 zur Längsachse des Innenteils 63 hin zunehmend zusammengebogen, was seine Reibungsfläche gegenüber der Innenwandung des Elektrodenkörpers und den Anpressdruck an diese erhöht, wodurch der weiteren Bewegung ein ansteigender Reibwiderstand entgegengesetzt wird.

Im Übrigen ist die Ausführung der Erfindung auch in einer Vielzahl von Abwandlungen und Konstruktionen der hier gezeigten Beispiele und weiter oben hervorgehobenen Aspekte der Erfindung möglich.

## Patentansprüche

1. Implantierbare Leitung (10 - 60), die einen hohlen Elektrodenkörper (11 - 61) mit einem freien Ende (11a - 61a) und ein nahe dem freien Ende im Elektrodenkörper angeordnetes und diesem gegenüber axial verschiebliches oder drehverschiebliches Innenteil (13 - 63) aufweist, das ein zum freien Ende des Elektrodenkörpers benachbartes Ende hat, an dem ein durch die Axialverschiebung des Innenteils aus dem freien Ende des Elektrodenkörpers ausfahrbares Fixierungsmittel (15 - 65) zur Fixierung der Leitung an Körpergewebe im implantierten Zustand angebracht ist, wobei am Innenumfang des Elektrodenkörpers ein elastisch deformierbares umfängliches, insbesondere ring- oder ringsegmentförmiges, Dicht- und/oder Widerstandselement (17 - 37) fixiert und am Außenumfang des Innenteils ein Abschnitt (13c, 23c, 23f; 33f, 33g, 33h) mit sich in axialer Richtung ändernden, insbesondere zum freien Ende des Elektrodenkörpers hin verringerndem Durchmesser vorgesehen ist, welcher bezüglich des Dicht- und Widerstandselementes derart platziert ist, dass er bei der Axialverschiebung des Innenteils das Dicht- und Widerstandselement passiert, oder
wobei am Außenumfang des Innenteils ein elastisch deformierbares ring- oder ringsegmentförmiges Dicht- und Widerstandselement (47 - 67) fixiert und am Innenumfang des Elektrodenkörpers ein Abschnitt (41d; 51d, 51e; 61d) mit sich in axialer Richtung ändernden, insbesondere zum freien Ende des Elektrodenkörpers hin verringerndem Durchmesser vorgesehen ist, welcher bezüglich des Dicht- und Widerstandselementes derart platziert ist, dass das Dicht- und Widerstandselement ihn bei der Axialverschiebung des Innenteils passiert,
wobei das Dicht- und Widerstandselement bei der Axialverschiebung des Innenteils zunehmend verformt wird und der Bewegung des Innenteils und somit dem Ausfahren des Fixierungsmittels zunehmenden Widerstand entgegensetzt.

2. Implantierbare Leitung (10 - 60) nach Anspruch 1, ausgebildet als Elektrodenleitung zum Anschluss an ein elektromedizinisches Gerät, mit mindestens einem Elektrodenpol und mindestens einer elektrischen Zuleitung.

3. Implantierbare Leitung nach Anspruch 1, ausgebildet als Katheterleitung.

4. Implantierbare Leitung (10 - 60) nach einem der vorangehenden Ansprüche, wobei das Innenteil (13 - 63) im Elektrodenkörper (11 - 61) drehverschieblich gehaltert ist, derart, dass das Fixierungsmittel (15 - 65) durch überlagerte Axialverschiebung und Rotation aus dem freien Ende des Elektrodenkörpers ausfahrbar ist.

5. Implantierbare Leitung nach Anspruch 4, wobei das Fixierungsmittel eine Schraubhelix (15 - 65) ist.

6. Implantierbare Leitung nach einem der vorangehenden Ansprüche, wobei das Dicht- und Widerstandselement (17 - 57) als Ring aus kompressiblem Material ist, welcher am Innenumfang des Elektrodenkörpers (11 - 31) oder am Außenumfang des Innenteils (43 - 53) derart eingespannt ist, dass er beim Passieren des Abschnitts mit veränderlichem Durchmesser des jeweils anderen der Teile Innenteil und Elektrodenkörper beim Ausfahren des Fixierungsmittels zunehmend komprimiert wird.

7. Implantierbare Leitung nach einem der vorangehenden Ansprüche, wobei das Dicht- und Widerstandselement (67) aus elastischem Material ist, welches am Innenumfang des Elektrodenkörpers (11 - 31) oder am Außenumfang des Innenteils (43 - 53) derart eingespannt und mit einer solchen geometrischen Konfiguration ausgebildet ist, dass es sich beim Passieren des Abschnitts veränderlichen Durchmessers des anderen der Teile Elektrodenkörper und Innenteil zum Ausfahren des Fixierungsmittels zunehmend und mit zunehmendem Widerstand deformiert.

8. Implantierbare Leitung nach Anspruch 6 oder 7, wobei das Dicht- und Widerstandselement (17 - 67) ein Silikon- oder Polyurethanmaterial aufweist.

9. Implantierbare Leitung nach einem der vorangehenden Ansprüche, wobei in axialer Richtung der Leitung am Elektrodenkörper oder Innenteil auf den Abschnitt mit sich änderndem Durchmesser (13c; 23c; 41d; 51d; 61d) mindestens einseitig ein Abschnitt konstanten Durchmessers folgt, dessen Durchmesser gleich dem kleinsten bzw. dem größten Durchmesser des Abschnitts mit sich verringerndem Durchmesser ist.

10. Implantierbare Leitung nach einem der vorangehenden Ansprüche, wobei an oder nahe einem Ende des Abschnitts (23c; 33g; 51d) mit sich änderndem Durchmesser ein Ringnut-Abschnitt (23f; 33f; 51e) angeordnet ist, in den das Dicht- und Widerstandselement (27; 37; 57) beim Ausfahren und/oder beim Wiedereinziehen des Fixierungsmittels (25; 35; 55) hineingleitet sich teilweise rück-verformt und einer weiteren axialen Bewegung des Innenteils (23; 33; 53) bezüglich des Elektrodenkörpers (21; 31; 51) einen sprunghaft erhöhten Widerstand entgegensetzt.

11. Implantierbare Leitung nach einem der vorangehenden Ansprüche, wobei der Abschnitt mit sich änderndem Durchmesser (13c; 23c; 33g; 61d) konisch ausgebildet ist.

12. Implantierbare Leitung nach einem der Ansprüche 1 - 10, wobei der Abschnitt (41d; 51 d) mit sich änderndem Durchmesser eine sich stufig oder bogenförmig verjüngende Oberfläche aufweist.

13. Implantierbare Leitung nach einem der vorangehenden Ansprüche, wobei das Innenteil (23; 33) oder der Elektrodenkörper (51) mehrere Abschnitte mit sich änderndem, insbesondere mit sich in entgegengesetzten Richtungen verringerndem, Durchmesser und/oder mit unterschiedlichem Kegelwinkel, aufweist.

14. Implantierbare Leitung nach einem der Ansprüche 9 bis 13, wobei auf mindestens einem Abschnitt des Innenteils (13 - 63) oder der Innenwandung des Elektrodenkörpers (11 - 61) eine Beschichtung mit vorbestimmtem Reibwert gegenüber dem Dicht- und Widerstandselement (17 - 67) zur selektiven Vergrößerung oder Verringerung des der axialen Verschiebung oder Drehverschiebung des Innenteils entgegengesetzten Widerstandes vorgesehen ist.

15. Implantierbare Leitung nach Anspruch 14, wobei auf verschiedenen Abschnitten des Innenteils (13 - 63) oder der Innenwandung des Elektrodenkörpers (11 - 61) Beschichtungen mit unterschiedlichem Reibwert gegenüber dem Dicht- und Widerstandselement vorgesehen sind.
